(19) Europäisches Patentamt
European Patent Office
Office européen des brevets

(11) Veröffentlichungsnummer: **0 135 062**
**B1**

(12) # EUROPÄISCHE PATENTSCHRIFT

(45) Veröffentlichungstag der Patentschrift: **25.11.87**

(51) Int. Cl.⁴: **C 07 D 319/06**, C 09 K 19/34

(21) Anmeldenummer: **84108781.0**

(22) Anmeldetag: **25.07.84**

(54) **Cyclohexyldioxane.**

(30) Priorität: **09.08.83 DE 3328638**

(43) Veröffentlichungstag der Anmeldung: **27.03.85 Patentblatt 85/13**

(45) Bekanntmachung des Hinweises auf die Patenterteilung: **25.11.87 Patentblatt 87/48**

(84) Benannte Vertragsstaaten: **AT CH DE FR GB IT LI NL**

(56) Entgegenhaltungen:
**EP-A-0 087 679**
**GB-A-2 044 767**
**GB-A-2 073 175**

(73) Patentinhaber: **Merck Patent Gesellschaft mit beschränkter Haftung, Frankfurter Strasse 250, D-6100 Darmstadt (DE)**

(72) Erfinder: **Krause, Joachim, Dr., Samuel- Morse-Strasse 14, D-6110 Dieburg (DE)**
Erfinder: **Eidenschink, Rudolf, Dr., Kornblumenstrasse 1, D-6115 Münster (DE)**
Erfinder: **Römer, Michael, Dr., Im Grossen Garten 18, D-6054 Rodgau 2 (DE)**
Erfinder: **Hittich, Reinhard, Dr., Am Kirchberg 11, D-6101 Modautal 1 (DE)**
Erfinder: **Weber, Georg, Wilhelm- Leuschner-Strasse 38, D-6106 Erzhausen (DE)**

**EP 0 135 062 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft neue Cyclohexyldioxanverbindungen der Formel I.

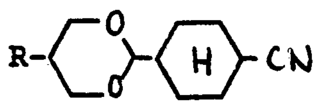

I

worin

R eine Alkylgruppe mit bis zu 12 C-Atomen, wobei 1 oder 2 $CH_2$-Gruppen durch O-Atome ersetzt sein können, bedeutet.

Die Verbindungen der Formel I können wie ähnliche Verbindungen als Komponenten flüssigkristalliner Dielektrika verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen. Ähnliche Verbindungen sind z. B. beschrieben im EP-A-87 679, GB-A-2 073 175 und GB-A-2 044 767.

Der Erfindung lag die Aufgabe zugrunde, neue stabile flüssigkristalline oder mesogene Verbindungen aufzufinden, die als Komponenten flüssigkristalliner Dielektrika geeignet sind. Diese Aufgabe wurde durch die Bereitstellung der Verbindungen der Formel I gelöst.

Es wurde gefunden, daß die Verbindungen der Formel I als Komponenten flüssigkristalliner Dielektrika vorzüglich geeignet sind. Insbesondere sind mit ihrer Hilfe stabile flüssigkristalline Phasen mit sehr niedriger optischer Anisotropie, positiver dielektrischer Anisotropie und vergleichsweise niedriger Viskosität herstellbar.

Mit der Bereitstellung der Verbindungen der Formel I wird außerdem ganz allgemein die Palette der flüssigkrislialline Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhägigkeit von der Auswahl der Substituenten können diese Verbindungen als Basismaterialien dienen, aus denen flüssigkristalline Dielektrika zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu senken. Die Verbindungen der Formel I eignen sich ferner als Zwischenprodukte zur Herstellung anderer Substanzen, die sich als Bestandteile flüssigkristalliner Dielektrika verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie ein Verfahren zu ihrer Herstellung, dadurch gekennzeichnet, daß man einen 4-Cyancyclohexancarbaldehyd oder eines seiner reaktionsfähigen Derivate mit 2-R-1,3-Propandiol umsetzt.

Weiterhin ist Gegenstand der Erfindung die Verwendung der Verbindungen der Formel I als Komponenten flüssigkristalliner Dielektrika. Gegenstand der Erfindung sind ferner flüssigkristalline Dielektrika mit einem Gehalt an mindestens einer Verbindung der Formel I sowie elektrooptische Anzeigeelemente, die derartige Dielektrika enthalten.

In den Verbindungen der Formel I sind diejenigen Stereoisomeren bevorzugt, worin die Substituenten an den Cyclohexylen- und den Dioxandiyl-Resten jeweils in trans-Stellung zueinander stehen.

Vor- und nachstehend hat die angegebene Bedeutung, sofern nicht ausdrücklich etwas anderes vermerkt ist.

In den Verbindungen der vor- und nachstehenden Formeln konnen die Alkylreste, in denen auch eine ("Alkoxy" bzw. "Oxaalkyl") oder zwei $CH_2$-Gruppen ("Alkoxyalkoxy bzw. Dioxaalkyl") durch O-Atome ersetzt sein können, geradkettig oder verzweigt sein. Vorzugsweise sind sie geradkettig, haben 2, 3, 4, 5, 6 oder 7 C-Atome und bedeuten dennach bevorzugt Ethyl, Propyl, Butyl, Pentyl, Hexyl, Heptyl, Ethoxy, Propoxy, Butoxy, Pentoxy, Hexoxy, Heptoxy, 2-Oxapropyl (= Methoxymethyl), 2-Oxabutyl (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, ferner Methyl, Octyl, Nonyl, Decyl, Methoxy, Octoxy, Nonoxy, Decoxy, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Verbindungen der Formeln I mit verzweigten Flügelgruppen R können gelegentlich wegen einer besseren Löslichkeit in den üblichen flüssigkristallinen Basismaterlalien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl.

Unter den Verbindungen der Formeln I sind diejenigen bevorzugt, in denen mindestens einer der darin enthaltenen Reste eine der angegebenen bevorzugten Bedeutungen hat.

Die Verbindungen der Formel I werden nach an sich bekannten Methoden hergestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Verbindung, die sonst der Formel I entspricht, aber an Stelle des Restes CN einen in CN umwandelbaren Rest Y trägt, in eine Verbindung der Formel I umwandelt.

Zur Herstellung von Nitrilen der Formel I können entsprechende Säureamide, z. B. solche, in denen an Stelle des Restes CN eine $CONH_2$-Gruppe steht, dehydratisiert werden. Die Amide sind z. B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$, $COCl_2$, ferner $P_2O_5$, $P_2S_5$, $AlCl_3$ (z. B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z. B. Basen wie Pyridin oder Triethylamin, aromatische Kohlenwasserstoffe wie Benzol, Toluol oder Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile isolieren.

Weiterhin kann man zur Herstellung der vorstehend genannten Nitrile der Formel I entsprechende N-Sulfohalogenid-Derivate von Amiden, vorzugsweise die N-Sulfochloride, in denen an Stelle des Restes X eine $CONHSO_2Cl$-Gruppe steht, einsetzen. Die Umwandlung in die Nitrile erfolgt zweckmäßig in einem aliphatischen oder aromatischen Kohlenwasserstoff als Lösungsmittel, besonders bevorzugt in Pentan, Hexan, Heptan, Benzol, Toluol oder Xylol, durch Behandeln mit Dimethylformamid (DMF), N-Methylpyrrolidon oder Dimethylacetamid bei Temperaturen zwischen etwa 0 und 50°, vorzugsweise bei Raumtemperatur. Nach üblicher Aufarbeitung kann man die Nitrile direkt isolieren. Die N-Sulfochlorid-Derivate von Amiden sind durch Umsetzung der entsprechenden Carbonsäuren mit Chlorsulfonylisocyanat erhältlich.

Zur Herstellung von Nitrilen der Formel I können auch entsprechende Chlor- oder Bromverbindungen mit einem Cyanid umgesetzt werden, zweckmäßig mit einem Metallcyanid wie NaCN, KCN oder $Cu_2(CN)_2$, z. B. in Gegenwart von Pyridin in einem inerten Lösungsmittel wie DMF oder N-Methylpyrrolidon bei Temperaturen zwischen 20° und 200°.

Verbindungen der Formel I sind weiterhin erhältlich, indem man einen 4-Cyancyclohexancarbaldehyd oder eines seiner reaktionsfähigen Derivate mit 2-R-1,3-Propandiol oder einem seiner reaktionsfähigen Derivate umsetzt, vorzugsweise in Gegenwart eines inerten Lösungsmittels wie Benzol oder Toluol und/oder eines Katalysators, z. B. einer starken Säure wie Schwefelsäure, Benzol-oder p-Toluolsulfonsäure, bei Temperaturen zwischen etwa 20° und etwa 150°, vorzugsweise zwischen 80° und 120°. Als reaktionsfähige Derivate der Ausgangsstoffe eignen sich in erster Linie Acetale z. B. der Formel $NC\text{-}Cy\text{-}CH(OR^1)_2$, worin $R^1$ Alkyl mit 1 - 4 C-Atomen, zwei Reste $R^1$ zusammen auch Alkylen mit 2 oder 3 C-Atomen bedeutet.

Die genannten Aldehyde und 1,3-Diole sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester erhältlich.

Die erfindungsgemäßen Dielektrika bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen aus den Klassen der Azoxybenzole, Benzylidenaniline, Biphenyle, Terphenyle, Phenyl- oder Cyclohexylbenzoate, Cyclohexancarbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexyldioxane, gegebenenfalls halogenierten Stilbene, Benzylphenylether, Tolane und substituierten Zimtsäuren.

Die wichtigsten als Bestandteile derartiger flüssigkristalliner Dielektrika in Frage kommenden Verbindungen lassen sich durch die Formel II charakterisieren,

$R^2\text{-}A\text{-}G\text{-}E\text{-}R^3$ II

worin A und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | -CH=CH- | -N(O)=N- |
| | -CH=CY- | -CH=N(O)- |
| | -C≡C- | $-CH_2-CH_2-$ |
| | -CO-O- | $-CH_2-O-$ |
| | -CO-S- | $-CH_2-S-$ |
| | -CH=N- | -COO-Phe-COO- |

oder eine C-C-Einfachbindung, Y Halogen, vorzugsweise Chlor, oder -CN, $R^2$ und $R^3$ Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, H, F, Cl oder Br und Phe 1,4-Phenylen bedeuten.

Bei den meisten dieser Verbindungen sind $R^2$ und $R^3$ voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich.

Die erfindungsgemäßen Dielektrika enthalten etwa 0,1 bis 90, vorzugsweise 5 bis 40 %, einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammoniumtetraphenylboranat oder Komplexsalze von Kronenethern (vergl. z. B. I. Haller et al., Mol. Cryst. Liq. Cryst. Band 24, Seiten 249 - 258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind zum Beispiel in den DE-OS-22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturen sind in Grad Celsius angegeben.

**Beispiel 1**

2,84 g trans-5-n-Pentyl-1,3-dioxan-2-yl-cyclohexan-4-carbonsäure löst man in 10 ml Benzol und tropft 1,5 g Chlorsulfonylisocyanat zu. Anschließend wird bis zum Ende der $CO_2$-Entwicklung auf 60° erwärmt. Nach dem Abkühlen auf Raumtemperatur gibt man 1,6 g Dimethylformamid zu und rührt noch 30 min. nach. Man gießt auf Eis, trennt die Benzolphase ab und erhält nach Trocknung und Abdestillieren des Lösungsmittels trans-2-(trans-4-Cyanocyclohexyl)-5-n-pentyl-1,3-dioxan.

Analog werden hergestellt:
trans-2-(trans-4-Cyanocyclohexyl)-5-methyl-1,3-dioxan
trans-2-(trans-4-Cyanocyclohexyl)-5-ethyl-1,3-dioxan
trans-2-(trans-4-Cyanocyclohexyl)-5-n-propyl-1,3-dioxan
trans-2-(trans-4-Cyanocyclohexyl)-5-n-butyl-1,3-dioxan
trans-2-(trans-4-Cyanocyclohexyl)-5-n-hexyl-1,3-dioxan
trans-2-(trans-4-Cyanocyclohexyl)-5-n-heptyl-1,3-dioxan
trans-2-(trans-4-Cyanocyclohexyl)-5-n-octyl-1,3-dioxan
trans-2-(trans-4-Cyanocyclohexyl)-5-n-nonyl-1,3-dioxan
trans-2-(trans-4-Cyanocyclohexyl)-5-n-decyl-1,3-dioxan
trans-2-(trans-4-Cyanocyclohexyl)-5-n-undecyl-1,3-dioxan
trans-2-(trans-4-Cyanocyclohexyl)-5-n-dodecyl-1,3-dioxan

**Beispiel 2**

Ein Gemisch von 2,5 g trans-4-Cyanocyclohexan-carbaldehyd (Synthese beschrieben in DE-OS-32 26 051), 2,9 g 2-n-Pentylpropan-1,3-diol, 0,01 g p-Toluolsulfonsäure und 20 ml Toluol wird am Wasserabscheider gekocht, abgekühlt, mit Wasser gewaschen und eingedampft. Man erhält nach üblicher chromatographischer Aufreinigung trans-2-(trans-4-Cyanocyclohexyl)-5-n-pentyl-1,3-dioxan.

Analog werden hergestellt:
trans-2-(trans-4-Cyanocyclohexyl)-5-ethoxy-1,3-dioxan
trans-2-(trans-4-Cyanocyclohexyl)-5-n-propoxy-1,3-dioxan
trans-2-(trans-4-Cyanocyclohexyl)-5-n-butoxy-1,3-dioxan

trans-2-(trans-4-Cyanocyclohexyl)-5-n-pentoxy-1,3-dioxan
trans-2-(trans-4-Cyanocyclohexyl)-5-n-hexoxy-1,3-dioxan
trans-2-(trans-4-Cyanocyclohexyl)-5-n-heptoxy-1,3-dioxan
trans-2-(trans-4-Cyanocyclohexyl)-5-(2-oxapropyl)-1,3-dioxan
trans-2-(trans-4-Cyanocyclohexyl)-5-(3-oxabutyl)-1,3-dioxan
trans-2-(trans-4-Cyanocyclohexyl)-5-(3-oxapentyl)-1,3-dioxan
trans-2-(trans-4-Cyanocyclohexyl)-5-(3-oxahexyl)-1,3-dioxan
trans-2-(trans-4-Cyanocyclohexyl)-5-(3-oxaheptyl)-1,3-dioxan
trans-2-(trans-4-Cyanocyclohexyl)-5-(1,3-dioxabutyl)-1,3-dioxan
trans-2-(trans-4-Cyanocyclohexyl)-5-(1,4-dioxapentyl)-1,3-dioxan
trans-2-(trans-4-Cyanocyclohexyl)-5-(1,4-dioxahexyl)-1,3-dioxan
trans-2-(trans-4-Cyanocyclohexyl)-5-(1,4-dioxaheptyl)-1,3-dioxan
trans-2-(trans-4-Cyanocyclohexyl)-5-isopropyl-1,3-dioxan
trans-2-(trans-4-Cyanocyclohexyl)-5-(1-methylpropyl)-1,3-dioxan.
(+)-trans-2-(trans-4-Cyanocyclohexyl)-5-(2-methylbutyl)-1,3-dioxan
(+)-trans-2-(trans-4-Cyanocyclohexyl)-5-(2-methylbutoxy)-1,3-dioxan

Es folgen Beispiele für erfindungsgemäße Dielektrika mit einem Gehalt an mindestens einer Verbindung der Formel I:

**Beispiel A**

Man stellt ein Gemisch her aus:
19 % p-trans-4-n-Propylcyclohexyl-benzonitril
15 % trans-4-n-Propylcyclohexancarbonsäure-(p-ethoxyphenylester)
14 % trans-4-n-Butylcyclohexancarbonsäure-(p-ethoxyphenylester)
17 % trans-4-n-Pentylcyclohexancarbonsäure-(p-methoxyphenylester)
18 % trans-4-n-Pentylcyclohexancarbonsäure-(p-n-pentylphenylester)
10 % trans-4-n-Butylcyclohexancarbonsäure-(p-trans-4-n-propyl-cyclohexylphenylester) und
7 % 2-(trans-4-n-Cyanocyclohexyl)-5-n-pentyl-1,3-dioxan.
F. < -10°, K. 64°, optische Anisotropie $\Delta n = 0{,}09$.

**Beispiel B**

In 98 Gewichtsteilen des Gemisches nach Beispiel A löst man 2 Gewichtsteile des blauen Farbstoffes 4,8-Diamino-1,5-dihydroxy-2-p-methoxyphenyl-anthrachinon.

**Beispiel C**

Man stellt ein Gemisch her aus:
19 % p-trans-4-n-Propylcyclohexyl-benzonitril
18 % p-trans-4-n-Pentylcyclohexyl-benzonitril
5 % 4-(trans-4-n-Pentylcyclohexyl)-4'-(trans-4-n-propylcyclohexyl)-biphenyl
19 % trans-4-n-Pentylcyclohexancarbonsäure-(p-n-pentylphenylester)
16 % trans-4-n-Propylcyclohexancarbonsäure-(p-ethoxyphenylester) und
23 % 2-(trans-4-Cyanocyclohexyl)-5-n-butyl-1,3-dioxan.
F. -10°, K. 60°.

**Beispiel D**

In 99,5 Gewichtsteilen des Gemisches nach Beispiel C löst man 0,5 Gewichtsteile Perylen-3,9-bis-carbonsäure-bis-(p-isopropyl-phenylester).

**Beispiel E**

Man stellt ein Gemisch her aus:
25 % trans-1-p-Ethoxyphenyl-4-n-propylcyclohexan
20 % 2-(trans-4-Cyanocyclohexyl)-5-ethyl-1,3-dioxan
20 % 2-(trans-4-Cyanocyclohexyl)-5-n-butyl-1,3-dioxan
10 % trans-4-n-Pentylcyclohexancarbonsäure-(p-trans-4-n-propylcyclohexylphenylester)
7 % trans,trans-4-n-Butylcyclohexylcyclohexan-4'-carbonsäure-trans-4-n-propylcyclohexylester und
18 % trans-4-n-Propylcyclohexancarbonsäure-(p-ethoxyphenylester).
Die flüssigkristalline Mischung hat eine kleine optische Anisotropie und ist vorzüglich für Multiplexbetrieb geeignet.

**Beispiel F**

In 99 Gewichtsteilen des Gemisches nach Beispiel E löst man 1 Gewichtsteil des roten Farbstoffes 1-p-Dimethylamino-benzylidenamino-4-p-cyanophenylazonaphthalin.

**Beispiel G**

Ein Gemisch aus
11,7 % trans,trans-4-Ethylcyclohexylcyclohexan-4'-carbonitril
6,3 % trans,trans-4-n-Propylcyclohexylcyclohexan-4'-carbonitril
13,8 % trans,trans-4-n-Butylcyclohexylcyclohexan-4'-carbonitril
6,5 % trans,trans-4-Pentylcyclohexylcyclohexan-4'-carbonitril
6,4 % 2-p-Cyanphenyl-5-propyl-1,3-dioxan
6,5 % 2-p-Cyanphenyl-5-pentyl-1,3-dioxan
4,4 % 4-Ethyl-4'-(trans-4-n-pentylcyclohexyl)-biphenyl
6,5 % p-Ethylbenzoesäure-(p-cyanphenylester)
8,6 % trans-4-n-Pentylcyclohexancarbonsäure-(p-trans-4-n-butylcyclohexylphenylester)
5,5 % trans,trans-4-n-Butylcyclohexylcyclohexan-4'-carbonsäure-trans-4-n-propylcyclohexylester
15,2 % trans-4-n-Pentylcyclohexancarbonsäure-(trans-4-n-propylcyclohexylester)
7,6 % r-1-Cyan-cis-4-(trans-4-n-propylcyclohexyl)-1-n-pentylcyclohexan
1,0 % 2-(trans-4-Cyanocyclohexyl)-5-n-octyl-1,3-dioxan
hat eine optische Anisotropie von $\Delta n = 0{,}10$.

**Beispiel H**

Man stellt ein Gemisch her aus:
16,4 % 2-(trans-4-Cyanocyclohexyl)-5-methyl-1,3-dioxan
11,9 % 2-(trans-4-Cyanocyclohexyl)-5-ethyl-1,3-dioxan
21,8 % 2-(trans-4-Cyanocyclohexyl)-5-n-pentyl-1,3-dioxan
17,4 % 2-(trans-4-Cyanocyclohexyl)-5-n-heptoxy-1,3-dioxan
10,8 % 2-(trans-4-Cyanocyclohexyl)-5-n-pentoxy-1,3-dioxan
10,9 % 2-(trans-4-Fluorcyclohexyl)-5-n-pentyl-1,3-dioxan
4,3 % 4-Ethyl-4'-cyanbiphenyl
6,5 % 4-p-Cyanphenyl-4'-n-pentyl-biphenyl.

**Patentansprüche** für die Vertragsstaaten: CH, DE, FR, GB, IT, LI, NL.

1. Verbindungen der Formel I

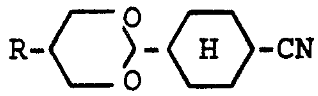

I

worin

R eine Alkylgruppe mit bis zu 12 C-Atomen, wobei eine oder zwei $CH_2$-Gruppen durch O-Atome ersetzt sein können,

bedeutet.

2. Verfahren zur Herstellung von Verbindungen der Formel I nach Anspruch 1, dadurch gekennzeichnet, daß man einen 4-Cyancyclohexancarbaldehyd oder eines seiner reaktionsfähigen Derivate mit 2-R-1,3-Propandiol umsetzt.

3. Verbindungen der Formel I nach Anspruch 1, worin R Methyl, Ethyl, Propyl, Butyl, Pentyl, Hexyl, Heptyl, Octyl, Nonyl, Decyl, Undecyl oder Dodecyl bedeutet.

4. Verbindungen der Formel I nach Anspruch 1, worin R Ethoxy, Propoxy, Butoxy, Pentoxy, Hexoxy, Heptoxy, 2-Oxapropyl, 3-Oxabutyl, 2-, 3- oder 4-Oxapentyl, 2-, 3-, 4- oder 5-Oxahexyl, 2-, 3-, 4-, 5-, 6-Oxaheptyl, 1,3-Dioxabutyl, 1,3-, 1,4- oder 2,4-Dioxapentyl, 1,3-, 1,4-, 1,5-, 2,4-, 2,5- oder 3,5-Dioxahexyl, oder 1,3-, 1,4-, 1,5-, 1,6-, 2,4-, 2,5-, 2,6-, 3,5-, 3,6- oder 4,6-Dioxaheptyl bedeutet.

5. Verwendung der Verbindungen der Formel I nach Anspruch 1 als Komponenten flüssigkristalliner Dielektrika für elektrooptische Anzeigeelemente.

6. Flüssigkristallines Dielektrikum für elektrooptische Anzeigeelemente mit mindestens zwei flüssigkristallinen Komponenten, dadurch gekennzeichnet, daß mindestens eine Komponente eine Verbindung der Formel 1 nach Anspruch 1 ist.

7. Elektrooptisches Anzeigeelement, dadurch gekennzeichnet, daß es ein Dielektrikum nach Anspruch 6 enthält.

**Patentansprüche** für den Vertragsstat: AT.

1. Verfahren zur Herstellung von Verbindungen der Formel I

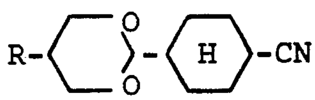

I

worin

R eine Alkylgruppe mit bis zu 12 C-Atomen, wobei eine oder zwei $CH_2$-Gruppen durch O-Atome ersetzt sein können,

bedeutet,

dadurch gekennzeichnet, daß man einen 4-Cyancyclohexancarbaldehyd oder eines seiner reaktionsfähigen Derivate mit 2-R-1,3-Propandiol umsetzt.

2. Verfahren zur Herstellung von Verbindungen der Formel I nach Anspruch 1, worin R Methyl, Ethyl, Propyl, Butyl, Pentyl, Hexyl, Heptyl, Octyl, Nonyl, Decyl, Undecyl oder Dodecyl bedeutet.

3. Verfahren zur Herstellung von Verbindungen der Formel I nach Anspruch 1, worin R Ethoxy, Propoxy, Butoxy, Pentoxy, Hexoxy, Heptoxy, 2-Oxapropyl, 3-Oxabutyl, 2-, 3- oder 4-Oxapentyl, 2-, 3-, 4- oder 5-Oxahexyl, 2-, 3-, 4-, 5-, 6-Oxaheptyl, 1,3-Dioxabutyl, 1,3-, 1,4- oder 2,4-Dioxapentyl, 1,3-, 1,4-, 1,5-, 2,4-, 2,5- oder 3,5-Dioxahexyl, oder 1,3-, 1,4-, 1,5-, 1,6-, 2,4-, 2,5-, 2,6-, 3,5-, 3,6- oder 4,6-Dioxaheptyl bedeutet.

4. Verwendung der Verbindungen der Formel I nach Anspruch 1 als Komponenten flüssigkristalliner Dielektrika für elektrooptische Anzeigeelemente.

5. Flüssigkristallines Dielektrikum für elektrooptische Anzeigeelemente mit mindestens zwei flüssigkristallinen Komponenten, dadurch gekennzeichnet, daß mindestens eine Komponente eine Verbindung der Formel I nach Anspruch 1 ist.

6. Elektrooptisches Anzeigeelement, dadurch gekennzeichnet, daß es ein Dielektrikum nach Anspruch 5 enthält.

**Claims** for the contracting States: CH, DE, FR, GB, IT, LI, NL.

1. Compounds of the formula

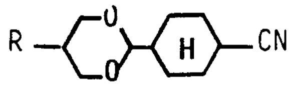
I

wherein R is an alkyl group having up to 12 C atoms in which 1 or 2 $CH_2$ groups can be replaced by O atoms.

2. Process for the preparation of compounds of the formula according to Claim 1, characterised in that a 4-cyano-cyclohexane-carbaldehyde or a reactive derivative thereof is reacted with 2-R-1,3-propanediol.

3. Compounds of the formula I, according to Claim 1, wherein R is methyl, ethyl, propyl, butyl, pentyl, hexyl, heptyl, octyl, nonyl, decyl, undecyl or dodecyl.

4. Compounds of the formula I, according to Claim 1, wherein R is ethoxy, propoxy, butoxy, pentoxy, hexoxy, heptyloxy, 2-oxapropyl, 3-oxabutyl, 2-, 3- or 4-oxapentyl, 2-, 3-, 4- or 5-oxahexyl, 2-, 3-, 4-, 5- or 6-oxaheptyl, 1,3-dioxabutyl, 1,3-, 1,4- or 2,4-dioxapetyl, 1,3-, 1,4-, 1,5-, 2,4-, 2,5- or 3,5-dioxahexyl or 1,3-, 1,4-, 1,5-, 1,6-, 2,4-, 2,5-, 2,6-, 3,5-, 3,6- or 4,6-dioxaheptyl.

5. The use of the compounds of the formula I, according to Claim 1, as components of liquid-crystal dielectrics for electrooptical display elements.

6. Liquid-crystal dielectric for electrooptical display elements having at least two liquid-crystal components, characterised in that at least one component is a compound of the formula I, according to Claim 1.

7. Electrooptical display element characterised in that it contains a dielectric according to Claim 6.

**Claims** the contracting State: AT.

1. Process for the preparation of compounds of the formula I

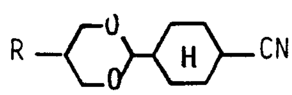
I

wherein R is an alkyl group having up to 12 C atoms in which 1 or 2 $CH_2$ groups can be replaced by O atoms, characterised in that a 4-cyano-cyclohexane-carbaldehyde or a reactive derivative thereof is reacted with 2-R-1,3-propanediol.

2. Process for the preparation of compounds of the formula I, according to Claim 1, wherein R is methyl, ethyl, propyl, butyl, pentyl, hexyl, heptyl, octyl, nonyl, decyl, undecyl or dodecyl.

3. Process for the preparation of compounds of the formula I, according to Claim 1, wherein R is ethoxy, propoxy, butoxy, pentoxy, hexoxy, heptyloxy, 2-oxapropyl, 3-oxabutyl, 2-, 3- or 4-oxapentyl, 2-, 3-, 4- or 5-oxahexyl, 2-, 3-, 4-, 5- or 6-oxaheptyl, 1,3-dioxabutyl, 1,3-, 1,4- or 2,4-dioxapentyl, 1,3-, 1,4-, 1,5-, 2,4-, 2,5-or 3,5-dioxahexyl or 1,3-, 1,4-, 1,5-, 1,6-, 2,4-, 2,5-, 2,6-, 3,5-, 3,6- or 4,6-dioxaheptyl.

4. The use of the compounds of the formula I, according to Claim 1, as components of liquid-crystal dielectrics for electrooptical display elements.

5. Liquid-crystal dielectric for electrooptical display elements having at least two liquid-crystal components, characterised in that at least one component is a compound of the formula I, according to Claim 1.

6. Electrooptical display element characterised in that it contains a dielectric according to Claim 5.

**Revendications** pour les Etats contractants: CH, DE, FR, GB, IT, LI, NL.

1. Composés de formule I

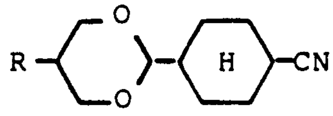
I

dans laquelle

R représente un groupe alkyle contenant jusqu'à 12 atomes de carbone et dans lequel un ou deux groupes $CH_2$ peuvent être remplacés par des atomes d'oxygène.

2. Procédé de préparation des composés de formule I selon la revendication 1, caractérisé en ce que l'on fait réagir un 4-cyanocyclohexane-carbaldéhyde ou l'un de ses dérivés réactifs avec le 2-R-1,3-propane-diol.

3. Composés de formule I selon la revendication 1, dans lesquels R représente un groupe méthyle, éthyle, propyle, butyle, pentyle, hexyle, heptyle, octyle, nonyle, décyle, undécyle ou dodécyle.

4. Composés de formule I selon la revendication 1, dans laquelle R représente un groupe éthoxy, propoxy, butoxy, pentoxy, hexoxy, heptoxy, 2-oxapropyle, 3-oxabutyle, 2-, 3- ou 4-oxapentyle, 2-, 3-, 4- ou 5-oxahexyle, 2-, 3-, 4-, 5-, 6-oxaheptyle, 1,3-dioxabutyle, 1,3-, 1,4-ou 2,4-dioxapentyle, 1,3-, 1,4-, 1,5-, 2,4-, 2,5- ou 3,5-dioxahexyle ou 1,3-, 1,4-, 1,5-, 1,6-, 2,4-, 2,5-, 2,6-, 3,5-, 3,6- ou 4,6-dioxaheptyle.

5. Utilisation des composés de formule I selon la revendication 1 en tant que composants de diélectriques à cristaux liquides pour des éléments d'affichage électro-optiques.

6. Diélectrique à cristaux liquides pour éléments d'affichage électro-optiques contenant au moins deux composants à cristaux liquides et caractérisé en ce que l'un au moins des composants est un composé de formule I selon la revendication 1.

7. Elément d'affichage électro-optique caractérisé en ce qu'il contient un diélectrique selon la rrevendication 6.

**Revendications** pour l'Etat contractant: AT.

1. Procédé de préparation des composés de formule I

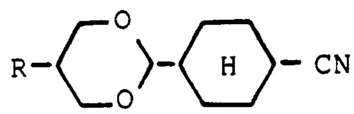

I

dans laquelle

R représente un groupe alkyle contenant jusqu'à 12 atomes de carbone et dans lequel un ou deux groupes $CH_2$ peuvent être remplacés par des atomes d'oxygène,

caractérisé en ce que l'on fait réagir un 4-cyanocyclohexane-carbaldéhyde ou l'un de ses dérivés réactifs avec le 2-R-1,3-propane-diol.

2. Procédé de préparation des composés de formule I selon la revendication 1 dans laquelle R représente un groupe méthyle, éthyle, propyle, butyle, pentyle, hexyle, heptyle, octyle, nonyle, décyle, undécyle ou dodécyle.

3. Procédé de préparation des composés de formule I selon la revendication 1 dans lesquels R représente un éthoxy, propoxy, butoxy, pentoxy, hexoxy, heptoxy, 2-oxapropyle, 3-oxabutyle, 2-, 3- ou 4-oxapentyle, 2-, 3-, 4- ou 5-oxahexyle, 2-, 3-, 4-, 5-, 6-oxaheptyle, 1,3-dioxabutyle, 1,3-, 1,4- ou 2,4-dioxapentyle, 1,3-, 1,4-, 1,5-, 2,4-, 2,5- ou 3,5-dioxahexyle ou 1,3-, 1,4-, 1,5-, 1,6-, 2,4-, 2,5-, 2,6-, 3,5-, 3,6- ou 4,6-dioxaheptyle.

4. Utilisation des composés de formule I selon la revendication 1 en tant que composants de diélectriques à cristaux liquides pour éléments d'affichage électro-optiques.

5. Diélectrique à cristaux liquides pour éléments d'affichage électro-optiques contenant au moins deux composants à cristaux liquides, caractérisé en ce que l'un au moins des composants est un composé de formule I selon la revendication 1.

6. Elément d'affichage électro-optique caractérisé en ce qu'il contient un diélectrique selon la revendication 5.